Europäisches Patentamt

European Patent Office

Office européen des brevets

(11) Veröffentlichungsnummer: **0 268 701**

**A1**

(12) **EUROPÄISCHE PATENTANMELDUNG**

(21) Anmeldenummer: 86116422.6

(22) Anmeldetag: 26.11.86

(51) Int. Cl.⁴ **A61N 1/36**

(43) Veröffentlichungstag der Anmeldung:
01.06.88 Patentblatt 88/22

(84) Benannte Vertragsstaaten:
**AT BE CH DE ES FR GB GR IT LI LU NL SE**

(71) Anmelder: **JAMUTRON GERÄTEBAU GmbH**
**Holbeinstrasse 4**
**D-8000 München 80(DE)**

(72) Erfinder: **Pfister, Stefan**
**Agnesstrasse 40**
**D-8000 München 40(DE)**

(74) Vertreter: **Füchsle, Klaus, Dipl.-Ing. et al**
**Hoffmann . Eitle & Partner Patentanwälte**
**Arabellastrasse 4**
**D-8000 München 81(DE)**

(54) **Reizstromgerät für medizinische und kosmetische Anwendung.**

(57) Die Erfindung betrifft ein Reizstromgerät für medizinische und kosmetische Anwendung. Ein Reizstromgenerator (G) zur Erzeugung von Reizstromimpulsen ist über steuerbare Schalter (SI, ...,S8) mit einer Anzahl von Behandlungsausgängen (Al,...,A8) verbunden. Eine elektronische Schaltersteuerung (St) schaltet die Schalter (Sl,..,S8) selektiv ein bzw. aus. Die Schaltersteuerung (St) enthält einen Taktsignalgenerator (Ts) und ein Programmteil (P), welches als Diodenmatrix-Schalter ausgebildet und leicht austauschbar ist. Hierdurch wird es möglich, die Behandlungsausgänge (Al,...,A8) nach einem beliebig vorgebbaren Behandlungsprogramm mit Reizstromimpulsen vom Reizstromgenerator (G) zu versorgen. Es eröffnen sich neue Behandlungsmethoden, insbesondere bei der Elektromassage und Elektroakupressur.

FIG. 1

## Reizstromgerät für medizinische und kosmetische Anwendung

Die Erfindung betrifft ein Reizstromgerät für medizinische uno kosmetische Anwendung, mit einem Reizstromgenerator zur Erzeugung von Reizstromimpulsen, und einer Anzahl von Behandlungsausgängen, die mit dem Reizstromgenerator verbunden sind und an die jeweils eine Körperelektrode anschließbar ist.

Die an ein derartiges Reizstromgerät angeschlossenen Körperelektroden werden dem Patienten an verschiedenen Stellen seines Körpers angelegt. Die in die Haut eingeleiteten Reizstromimpulse bewirken eine gezielte Stimulierung der Gewebepartien im Bereich der Elektroden. Je nach Höhe und Polarität der angelegten Spannung, Stromstärke sowie Impulsdauer und Impulshäufigkeit kommt es zu einer Förderung der lokalen Durchblutung bis hin zu gezielten Muskelbewegungen. Typische Anwendungen sind Massage, Elektro-Akupressur, kosmetische Haut-und Gewebestraffung, Figurtraining und Schlankheitsbehandlung.

Bei den bisher bekannten Reizstromgeräten ist nachteilig, daß alle vorhandenen Behandlungsausgänge mit den vom Reizstromgenerator erzeugten Reizstromimpulsen gleichermaßen versorgt werden, so daß alle Körperpartien, an denen Körperelektroden angelegt sind, immer nur gleichzeitig stimuliert werden können. Bei vielen Anwendungen, insbesondere Massage-und Akupressur-Behandlungen, ist aber die Einhaltung einer bestimmten zeitlichen Reihenfolge, in der einzelne Körperpunkte stimuliert werden, von großer Bedeutung für den Behandlungserfolg. So soll beispielsweise in der Regel von den Extremitäten zur Körpermitte, also zum Herzen hin massiert werden. Eine zeitliche Abfolge bei der Stimulierung bestimmter Körperpunkte läßt sich mit herkömmlichen Reizstromgeräten nur durch umständliches An-und Ablegen der Körperelektroden erreichen. Bei der Akupressur ist eine periodische Stimulierung einzelner Akupunkturpunkte in einer vorgegebenen Reihenfolge überhaupt nicht möglich, da diese Punkte vor jedem Anlegen einer Elektrode erst genau lokalisiert werden müssen.

Aufgabe vorliegender Erfindung ist es deshalb, ein Reizstromgerät zu schaffen, mit dem über angeschlossene Körperelektroden verschiedene Stellen des Körpers des Patienten in einer gewünschten zeitlichen Reihenfolge automatisch stimuliert werden können.

Bei der Lösung dieser Aufgabe wird ausgegangen von einem Reizstromgerät der eingangs genannten Art; gelöst wird die Aufgabe dadurch, daß zwischen jedem Behandlungsausgang und dem Reizstromgenerator ein steuerbarer Schalter angeordnet ist, und dadurch, daß eine elektronische Schaltersteuerung zum selektiven Ein-und Ausschalten der Schalter vorgesehen ist. Die von der elektronischen Schaltersteuerung angesteuerten Schalter legen den Ausgang des Reizstromgenerators selektiv an einen oder mehrere der Behandlungsausgänge, an welche die Körperelektroden angeschlossen sind. Auf diese Weise können die einzelnen Körperelektroden der Reihe nach mit Reizstromimpulsen versorgt werden, so daß verschiedene Stellen am Körper des Patienten in einer genau festgelegten Rehenfolge, welche sich auch beliebig oft wiederholen läßt, stimuliert werden können. Mit dem erfindungsgemäß ausgebildeten Reizstromgerät lassen sich deshalb insbesondere Elektromassagen und Akupressur-Behandlungen wesentlich wirksamer durchführen.

In vorteilhafter Weiterbildung des erfindungsgemäßen Reizstromgeräts enthält die Schaltersteuerung ein Programmteil, das die Reihenfolge des Ein-und Ausschaltens der Schalter zwischen den Behandlungsausgängen und dem Reizstromgenerator bestimmt. Mittels des Programmteils lassen sich spezifische Behandlungsprogramme für verschiedene Anwendungsfälle festlegen, die dann jederzeit abrufbar zur Verfügung stehen. Eine Anpassung des Reizstromgeräts an den jeweiligen Anwendungsfall ist so leicht möglich, da Änderungen lediglich im Programmteil vorgenommen werden müssen; ein Eingriff in die übrige Schaltersteuerung ist hierzu nicht erforderlich. Die dem jeweiligen Anwendungsfall gemäße Reihenfolge des Ein-und Ausschaltens der Schalter kann beispielsweise durch eine festverdrahtete Schaltung innerhalb des Programmteils niedergelegt sein. Es ist aber auch eine Lösung denkbar, bei der diese Reihenfolge softwaremäßig im Programmteil gespeichert ist.

Bei einer bevorzugten Ausführung der Erfindung enthält die Schaltersteuerung einen Taktsignalgeber zur Erzeugung periodischer Taktsignale. Die Taktsignale bestimmen die Einschaltzeiten der Schalter und damit die Zeiträume, in denen Reizstromimpulse an die Behandlungsausgänge bzw. Körperelektroden geleitet werden. Zweckmäßig weist der Taktsignalgeber Taktsignalkanäle in einer Anzahl auf, die der Anzahl der zu steuernden Schalter entspricht, wobei jeweils nur an einem dieser Taktsignalkanäle ein Taktsignal anliegt. Die Reihenfolge des Ein-und Ausschaltens der den Behandlungsausgängen zugeordneten Schaltern kann dann auf einfache Weise durch eine im Programmteil enthaltene Logikschaltung realisiert werden, welche die einzelnen Taktsignalkanäle des Taktsignalgebers den Schaltern selektiv zuordnet. Liegt beispielsweise an einem der Taktsignalkanäle des Taktsignalgebers ein Taktsignal an und verbindet die Logikschaltung diesen Taktsignalkanal mit einem bestimmten Schalter, so werden Reizstromimpulse von Reizstromgenerator an den zugeordneten Behandlungsausgang solange geleitet, wie das Taktsignal andauert. Anschließend wird ein anderer, von der Logikschaltung im Programmteil ausgewählter Behandlungsausgang mit Reizstromimpulsen versorgt.

Bevorzugt wird eine Ausführung, bei der das Programmteil jedem Taktsignalkanal mehrere, insbesondere zwei Schalter zuordnet. Damit lassen sich zwei oder auch mehrere Stellen am Körper des Patienten gleichzeitig stimulieren.

In zweckmäßiger Weiterbildung des Erfindungsgegenstands ist das Programmteil über eine lösbare Steckverbindung mechanisch und elektrisch mit der Schaltersteuerung verbunden. Dies ermöglicht eine leichte Austauschbarkeit des Programmteils, wodurch sich das Gerät besonders - schnell und einfach an verschiedene Anwendungsfälle anpassen läßt. Das austauschbare Programmteil enthält das für die jeweilige Behandlungsart am besten geeignete Behandlungsprogramm in Form einer vorgegebenen Reihenfolge des Ein- und Ausschaltens der den einzelnen Behandlungsausgängen zugeordneten Schaltern.

Bei einer besonders vorteilhaften Ausführung der Erfindung enthält das Programmteil eine Diodenmatrix-Schaltung. Die Zuordnung der zu steuernden Schalter zu den einzelnen Taktsignalkanälen des Taktsignalgebers erfolgt hierbei durch Dioden, welche an entsprechenden Stellen in die Verbindungsleitungen zwischen Taktsignalgeber und Schalter angeordnet sind. Unter Ausnutzung ihrer spezifischen Eigenschaft, daß sie den Stromfluß nur in der einen Richtung hindurchlassen, in der Gegenrichtung jedoch sperren, kann mit einer derartigen Diodenmatrix-Schaltung nahezu jede gewünschte Reihenfolge des Ein-und Ausschaltens der Schalter im Programmteil festgelegt werden. Insbesondere ist es leicht möglich, mehrere Schalter über den gleichen Taktsignalkanal anzusteuern, so daß zwei oder auch mehrere, beliebig auswählbare Behandlungsausgänge gleichzeitig mit Reizstromimpulsen versorgt werden. Diodenmatrix-Schaltungen lassen sich einfach und schnell herstellen, wobei auf standardisierte Bauelemente zurückgegriffen werden kann. Werden diese zudem in ein Programmteil eingesetzt, welches über eine leicht zugängliche Steckverbindung mit der Schaltersteuerung verbunden ist, so verfügt das Reizstromgerät über eine Art Programmstecker, der mit einem Handgriff ausgewechselt werden kann, um so ein anderes Behandlungsprogramm einzustellen. Ein Eingriff in das Geräteinnere ist hierzu nicht erforderlich.

In vorteilhafter Weiterbildung des erfindungsgemäßen Reizstromgeräts ist der Taktsignalgeber abschaltbar und ist ein Handschalter vorgesehen, mit dem sich einzelne Dauersignale auf einen der Taktsignalkanäle geben lassen. Unabhängig von der vorgegebenen Zeitsteuerung durch den Taktsignalgeber lassen sich so Reizstromimpulse für eine beliebig lange Zeitdauer an die Behandlungsausgänge leiten, wobei die Reihenfolge des Ein- und Ausschaltens der Schalter nach wie vor vom Programmteil vorgegeben ist, das Umschalten auf den nächsten Takt im Programmzyklus jedoch von Hand erfolgt. Diese Betriebsart ist insbesondere zur Geräteüberprüfung sowie zu Testzwecken vorteilhaft.

Zweckmäßigerweise sind die steuerbaren Schalter als Kontaktrelai ausgebildet. In diesem Fall werden die Taktsignale, gegebenenfalls nach entsprechender Verstärkung, auf die Erregerwicklungen gegeben, während die Schaltkontakte den Stromfluß vom Reizstromgenerator zu den Behandlungsausgängen unterbrechen. Es können auch Halbleiterschalter, beispielsweise Thyristoren, Verwendung finden.

Bei der Elektromassage und Elektroakupressur werden in der Regel zweipolige Körperelektroden verwendet. Aus Sicherheitsgründen ist es deshalb vorteilhaft, wenn die Behandlungsausgänge durch die Schalter zweipolig zu-bzw. abgeschaltet werden.

Ein Ausführungsbeispiel der Erfindung wird nachstehend anhand der beigefügten Zeichnungen näher erläutert. Es zeigen:

Fig. I ein Reizstromgerät mit 8 Behandlungsausgängen, in einem stark vereinfachten Prinzipbild,

Fig. 2 den Rechteckgenerator von Figur I, in einem Schaltbild;

Fig. 3 die Schaltersteuerung von Figur I, in einem Schaltbild;

Fig. 4 die Eingangs-und die Ausgangsseite des Programmteils von Figur 3;

Fig. 5 das als Dioden-Matrix ausgebildete Programmteil von Figur 3, in einem Schaltbild;

Fig. 6 die vom Taktsignalgeber von Figur 3 abgegebenen Taktsignale, welche auf den einzelnen Taktsignalkanälen anliegen, in einem Zeitdiagramm; und

Fig. 7 die Einschaltzeiten der von den Taktsignalen gemäß Figur 6 angesteuerten Schalter von Figur I.

Das Reizstromgerät gemäß Figur I hat insgesamt 8 Behandlungsausgänge AI bis A8, von denen hier lediglich die ersten beiden Ausgänge AI, A2 sowie der letzte Ausgang A8 dargestellt sind. Jeder dieser Behandlungsausgänge AI bis A8

ist über die Schaltkontakte eines zugehörigen Schaltrelais Sl bis S8 mit einem Reizstromgenerator G verbunden. An die Behandlungsausgänge Al bis A8 sind zweipolige Körperelektroden E angeschlossen, welche dem Patienten an verschiedenen Stellen seines Körpers angelegt werden. Die Erregerwicklungen der Schaltrelais Sl bis S8 werden von einer elektronischen Schaltersteuerung St angesteuert. Diese bewirkt ein selektives Ein-bzw. Ausschalten der Schaltrelais Sl bis S8, wodurch einer oder mehrerer der Behandlungsausgänge Al bis A8 mit Reizstromimpulsen vom Reizstromgenerator G versorgt werden. Die Schaltersteuerung St enthält einen Taktsignalgenerator Ts, welcher Taktsignale zum periodischen Umschalten der Schaltrelais Sl bis S8 abgibt. Ein ebenfalls in der Schaltsteuerung St enthaltenes Programmteil P ordnet die vom Taktsignalgenerator Ts abgegebenen Taktsignale den einzelnen Schaltrelais Sl bis S8 zu und bestimmt so die Reihenfolge deren Ein-und Ausschaltens. Die an die Behandlungsausgänge Al bis A8 angeschlossenen Körperelektroden E werden somit gemäß einer von der Schaltersteuerung St vorgegebenen zeitlichen Abfolge mit Reizstromimpulsen beaufschlagt.

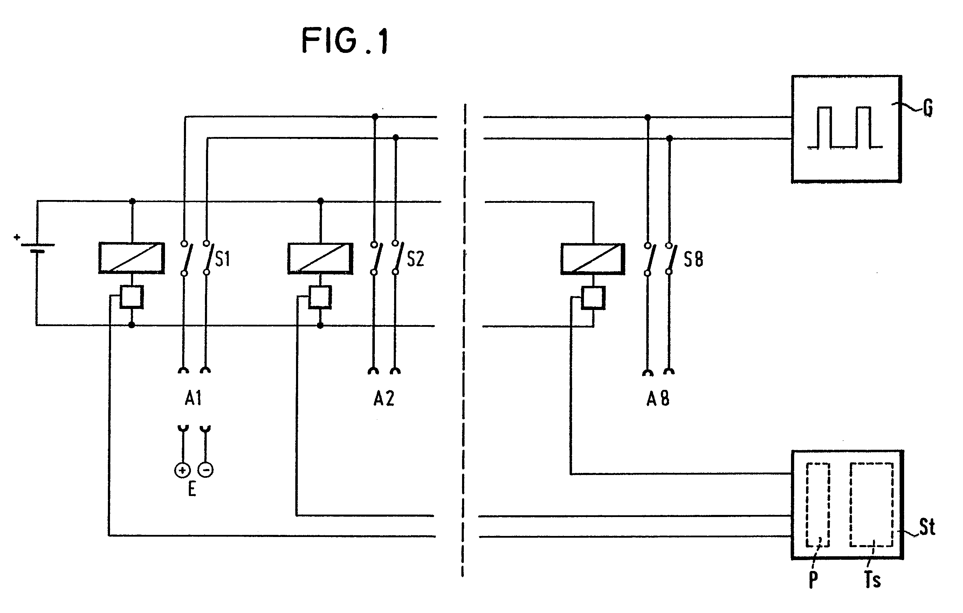

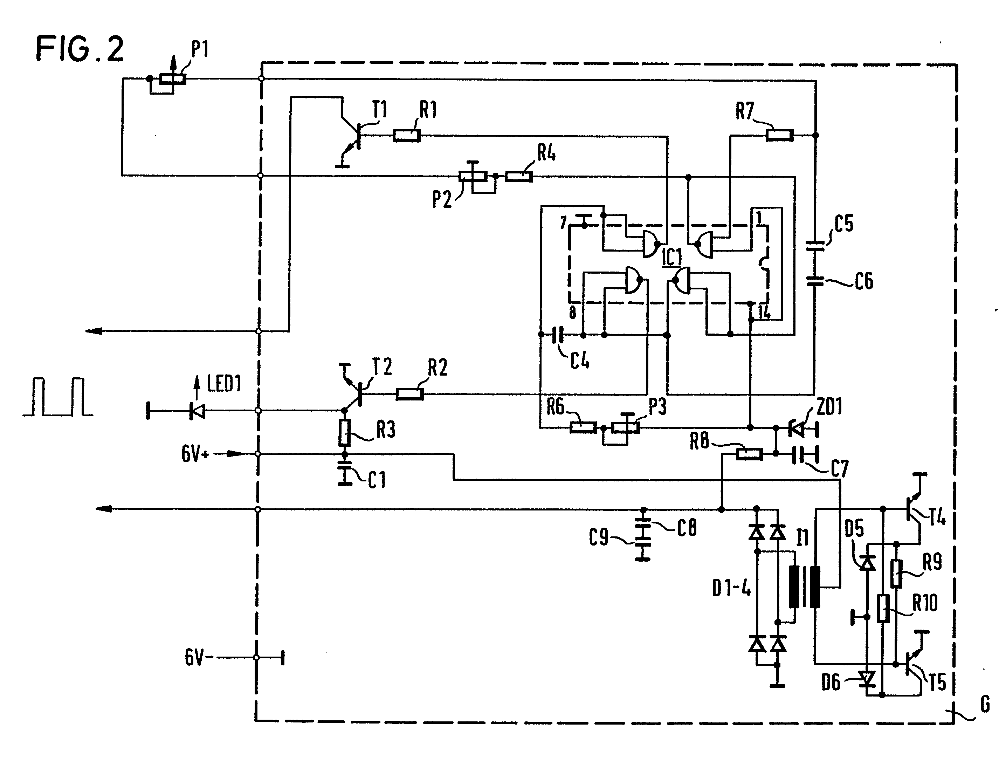

Fig. 2 zeigt den Reizstromgenerator G in einem detaillierten Schaltbild. Kernstück ist ein integrierter Schaltkreis ICl, welcher einen Multivibrator enthält. Es ist eine Gleichspannungsversorgung von 6 Volt vorgesehen. Am Ausgang wird Reizstrom in Form von Rechteckimpulsen abgegeben. Die Spitzenspannung beträgt ungefähr 50 Volt, die Impulsbreite beträgt zwischen 2 und 3 Millisekunden. Spitzenwert der vom Reizstromgenerator G abgegebenen Rechteckspannung sowie deren Frequenz sind mittels Potentiometer Pl und P2 einstellbar.

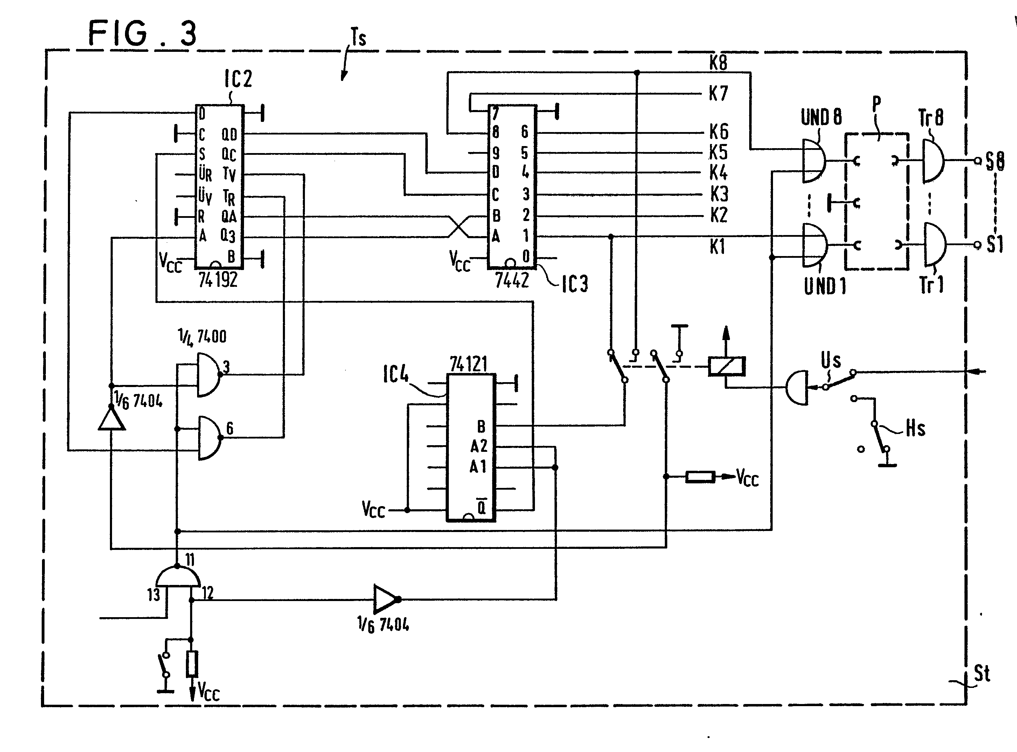

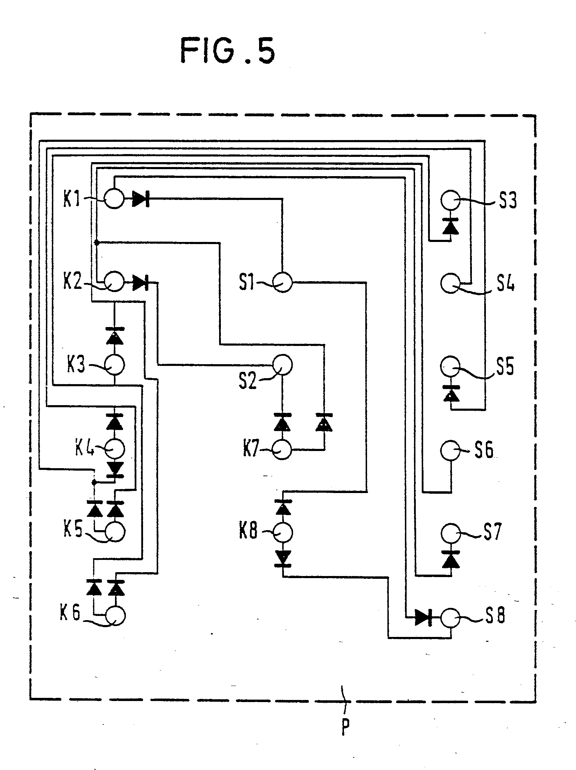

Die Schaltersteuerung St, welche in Figur 3 detailliert dargestellt ist, ist ebenfalls mit integrierten Schaltkreisen IC2, IC3 und IC4 aufgebaut. IC3 ist ein Dekadenzähler, der von IC2 angesteuert wird. IC4 dient der Erzeugung von Rücksetzsignalen. IC3 liefert über seine Zählausgänge eine periodische Folge von Taktsignalen, welche der Reihe nach an 8 Taktsignalkanälen Kl bis K8 anliegen. Über UND-Gatter UNDl bis UND8 sind die Taktsignalkanäle Kl bis K8 mit dem Programmteil P verbunden. In Figur 3 sind lediglich zwei UND-Gatter, nämlich UNDl, UND8, sowie die zugehörigen Verbindungsleitungen zu den Taktsignalkanälen Kl, K8 bzw. zum Programmteil P eingezeichnet. Nach Zuordnung der Taktsignalkanäle Kl bis K8 innerhalb des Programmteils P (dessen Schaltung später anhand von Figur 5 erläutert wird) und Verstärkung in nachgeschalteten Treiberstufen Trl bis Tr8 stehen an den acht separaten Ausgängen der Schaltersteuerung St Steuersignale zur Ansteuerung der Schaltrelais Sl bis S8 zur Verfügung. Ein Umschalter U dient zum Abschalten des Taktsignalgenerators Ts. Mittels eines Handschalters H lassen sich dann einzelne Dauersignale auf je einen der Taktsignalkanäle Kl bis K8 geben, wodurch die Schaltrelais Sl bis S8 der Reihe nach von Hand ansteuerbar sind.

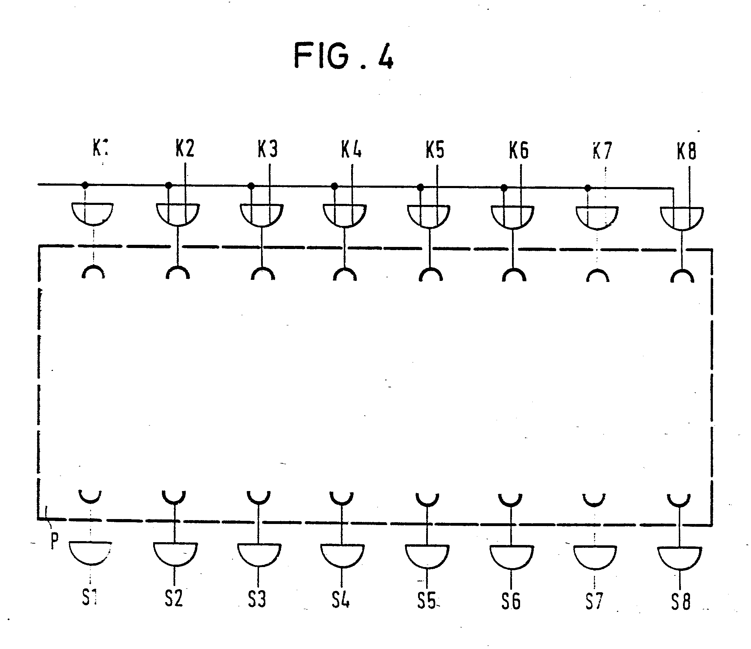

Fig. 4 verdeutlicht die Zusammenschaltung des Programmteils P eingangsseitig mit den acht Taktsignalkanälen Kl bis K8 und ausgangsseitig mit den Schaltrelais Sl bis S8 für die acht Behandlungsausgänge Al bis A8 (vgl. Figur l). Das Programmteil P ist von der übrigen Schaltersteuerung St körperlich getrennt und mit dieser über eine l6-polige Steckverbindung mechanisch und elektrisch verbunden.

Fig. 5 zeigt die Schaltung innerhalb des Programmteils P; sie ist hier als festverdrahtete Diodenmatrix-Schaltung ausgeführt. Die insgesamt l6, von außen zugänglichen Anschlüsse dieser Diodenmatrix-Schaltung sind in eine Gruppe von acht Eingangsanschlüssen, welche den Taktsignalkanälen Kl bis K8 entsprechen, und eine Gruppe von acht Ausgangsanschlüssen, welche den zu steuernden Schaltrelais Sl bis S8 zugeordnet sind, aufgeteilt. Jeder Eingang Kl bis K8 ist über Verbindungsleitungen und zwischengeschaltete Dioden mit genau zwei Ausgängen Sl bis S8 verbunden, unter Beachtung der Durchlaßrichtungen der Dioden. Die auf diese Weise realisierte Zuordnung der acht Taktsignalkanäle Kl bis K8 zu den einzelnen Schaltrelais Sl bis S8 ergibt sich aus nachstehender Zuordnungstabelle:

Kl: Sl, S8
K2: S2, S7
K3: S3, S6
K4: S4, S5
K5: S4, S5
K6: S3, S6
K7: S2, S7
K8: Sl, S8

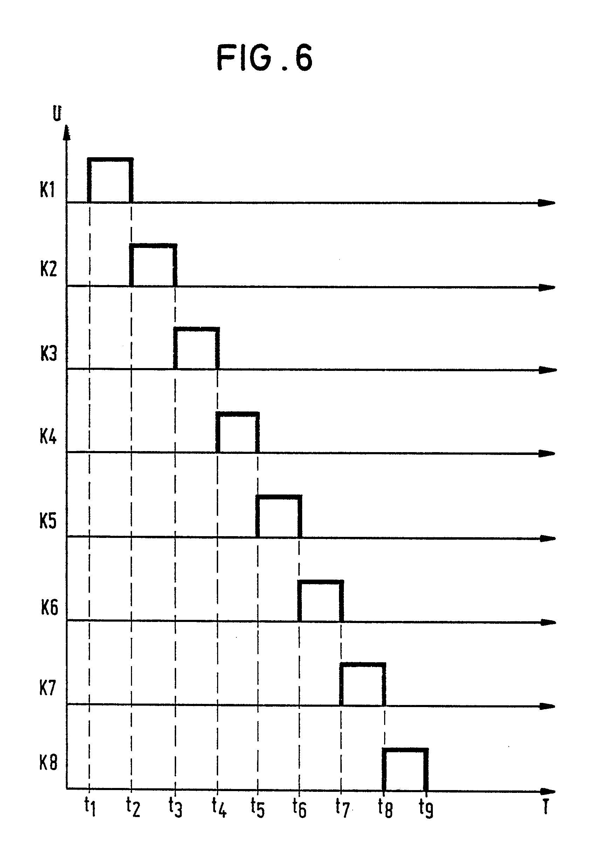

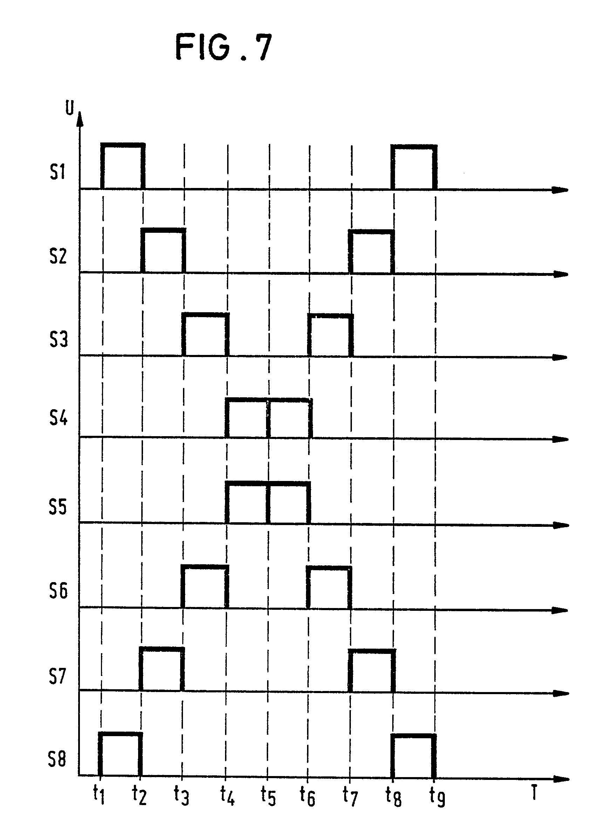

Die Funktion der Schaltersteuerung St und insbesondere das Zusammenwirken des Taktsignalgenerators Ts mit dem Programmteil P wird nun anhand der in den Figuren 6 und 7 dargestellten Zeitdiagramme erläutert.

Figur 6 gibt die an den Taktsignalkanälen Kl bis K8 anliegenden Taktsignale wieder. Zwischen den Zeitpunkten $t_1$ und $t_2$ liegt lediglich auf dem Taktsignalkanal Kl ein Taktsignal in Form einer Gleichspannung an. Zum Zeitpunkt $t_2$ wird das Taktsignal auf den Taktsignalkanal 2 umgeschaltet usw., bis zum Zeitpunkt $t_8$ an K8 ein Taktsignal anliegt. Anschließend wiederholt sich dieselbe Taktsignalfolge wieder, beginnend bei einem Taktsignal auf Kl zum Zeitpunkt $t_9$, welcher dem Zeitpunkt $t_1$ in Figur 6 entspricht.

Aufgrund der Zuordnung der einzelnen Taktsignalkanäle KI bis K8 zu den einzelnen Schaltrelais SI bis S8 durch die Diodenmatrix-Schaltung gemäß Figur 5 ergeben sich die in dem Zeitdiagramm von Figur 7 dargestellten Einschaltzeiten, während denen Heizstromimpulse vom Heizstromgenerator G zu den einzelnen Behandlungsausgängen AI bis A8 gelangen (vgl. Figur I). Gemäß der obigen Zuordnungstabelle sind zu einem beliebigen Zeitpunkt $t_1$ bis $t_2$ stets die Schaltkontakte von zwei der Schaltrelais SI bis S8 eingeschaltet. Beispielsweise werden im Zeitraum zwischen den Zeitpunkten $t_3$ und $t_4$ die Behandlungsausgänge A3 und A6 über die zugeordneten durchgeschalteten Schaltrelais S3 und S6 mit Reizstromimpulsen versorgt. Figur 7 gibt deshalb gleichzeitig ein Zeitdiagramm für die Stimulierung der verschiedenen Stellen des Körpers des Patienten wieder, an welchen die an die Behandlungsausgänge AI bis A8 angeschlossenen Körperelektroden E angelegt sind.

Die Diodenmatrix-Schaltung gemäß Figur 5 und die sich hieraus ergebende spezielle zeitliche Reihenfolge der Versorgung der Behandlungsausgänge AI bis A8 mit Reizstromimpulsen, wie in Figur 7 dargestellt, ist lediglich ein Beispiel dafür, in welcher Weise die Schaltrelais SI bis S8 von der Schaltersteuerung St selektiv angesteuert werden können. Mittels anders geschalteter Dioden in der Diodematrix-Schaltung von Figur 5 lassen sich leicht beliebige andere Schaltfolgen realisieren. Durch einfachen Austausch des als Programmstecker ausgebildeten Programmteils P liefert das Reizstromgerät so das für den jeweiligen Anwendungsfall am besten geeignete Behandlungsprogramm.

## Ansprüche

I. Reizstromgerät für medizinische und kosmetische Anwendung, mit
-einem Reizstromgenerator (G) zur Erzeugung von Reizstromimpulsen, und
-einer Anzahl von Behandlungsausgängen (AI,...,A8), die mit dem Reizstromgenerator (G) verbunden sind und an die jeweils eine Körperelektrode (E) anschließbar ist,

dadurch gekennzeichnet, daß

-zwischen jedem Behandlungsausgang (AI,...,A8) und dem Reizstromgenerator (G) ein steuerbarer Schalter (SI,...,S8) angeordnet ist, und
-eine elektronische Schaltersteuerung (St) zum selektiven Ein-und Ausschalten der Schalter (SI,...,S8) vorgesehen ist.

2. Reizstromgenerator nach Anspruch I, dadurch gekennzeichnet, daß die Schaltersteuerung (St) ein Programmteil (P) enthält, das die Reihenfolge des Ein-und Ausschaltens der Schalter (SI,...,S8) bestimmt.

3. Reizstromgenerator nach Anspruch I oder 2, dadurch gekennzeichnet, daß die Schaltersteuerung (St) einen Taktsignalgeber (Ts) zur Erzeugung periodischer Taktsignale enthält.

4. Reizstromgenerator nach Anspruch 5, dadurch gekennzeichnet, daß
-der Taktsignalgeber (Ts) Taktsignalkanäle (KI,...,K8) in einer Anzahl aufweist, die der Anzahl der zu steuernden Schalter (SI,...,S8) entspricht, und
-jeweils nur an einem dieser Taktsignalkanäle (KI,...,K8) ein Taktsignal anliegt.

5. Reizstromgenerator nach Anspruch 4, dadurch gekennzeichnet, daß das Programmteil (P) eine Logikschaltung enthält, welche die einzelnen Taktsignalkanäle (KI,...,K8) des Taktsignalgebers (Ts) den Schaltern (SI,...,S8) selektiv zuordnet.

6. Reizstromgenerator nach Anspruch 5, dadurch gekennzeichnet, daß das Programmteil (P) jedem Taktsignalkanal (KI,...,K8) mehrere, insbesondere zwei Schalter (SI,...,S8) zuordnet.

7. Reizstromgenerator nach Anspruch 5 oder 6, dadurch gekennzeichnet, daß das Programmteil (P) über eine lösbare Steckverbindung mechanisch und elektrisch mit der Schaltersteuerung (St) verbunden ist.

8. Reizstromgenerator nach einem der Ansprüche 5 bis 7, dadurch gekennzeichnet, daß das Programmteil (P) eine Diodenmatrix-Schaltung enthält.

9. Reizstromgenerator nach einem der Ansprüche 3 bis 8, dadurch gekennzeichnet, daß
-der Taktsignalgeber (Ts) abschaltbar ist, und
-ein Handschalter (H) vorgesehen ist, mit dem sich einzelne Dauersignale auf je einen der Taktsignalkanäle (KI,...,K8) geben lassen.

I0. Reizstromgenerator nach einem der Ansprüche I bis 9, dadurch gekennzeichnet, daß die Schalter (SI,...,S8) als Kontaktrelais ausgebildet sind.

II. Reizstromgenerator nach einem der Ansprüche I bis I0, dadurch gekennzeichnet, daß die Schalter (SI,...,S8) die Behandlungsausgänge (AI,...,A8) zweipolig zu-bzw. abschalten.

# FIG.1

0 268 701

FIG.2

0 268 701

44 857

FIG. 3

# FIG . 4

K1  K2  K3  K4  K5  K6  K7  K8

P

S1  S2  S3  S4  S5  S6  S7  S8

0 268 701

# FIG.5

# FIG.6

# FIG . 7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-2 437 346 (THOMA)<br><br>* Patentanspruch 1; Figur * | 1-3,5, 8,10, 11 | A 61 N 1/36 |
| | --- | | |
| X | EP-A-0 057 561 (BIOMEDICAL RESEARCH LTD)<br>* Patentansprüche 1-3,5; Figuren 1,2 * | 1-4 | |
| | --- | | |
| X | DE-A-3 318 874 (DATRON-ELEKTRONIK)<br>* Patentansprüche 11-13 * | 1-3,5 | |
| | --- | | |
| X | US-A-4 390 023 (RISE)<br>* Patentansprüche 1-5 * | 1-6,11 | |
| | --- | | RECHERCHIERTE SACHGEBIETE (Int. Cl.4) |
| X | DE-A-3 236 756 (PHYSIOMED-MEDIZINTECHNIK)<br>* Seite 7, Zeile 18 - Seite 8, Zeile 4; Zeichnung 1 * | 1,3,10 ,11 | A 61 N |
| | ---     -/- | | |

Der vorliegende Recherchenbericht wurde für alle Patentansprüche erstellt.

| Recherchenort<br>DEN HAAG | Abschlußdatum der Recherche<br>29-06-1987 | Prüfer<br>SCHMIERER U.J. |
|---|---|---|

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| X | IEEE TRANSACTIONS ON BIOMEDICAL ENGINEERING, Band BME-26, Nr. 2, Februar 1979, Seiten 112-116, IEEE, New York, US; P. STROJNIK et al.: "Programmed six-channel electrical stimulator for complex stimulation of leg muscles during walking" * Seite 114, Abschnitt E; Figur 2 * | 1-6 | |
| | --- | | |
| A | FR-A-2 504 807 (MEDTRONIC) * Seite 5, Zeilen 26-31; Figur 2 * | 1 | |
| | --- | | |
| A | FR-A-2 433 950 (INSTITUT DE CREATION ET D'ETUDES TECHNIQUES) | | |
| | ----- | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| DEN HAAG | 29-06-1987 | SCHMIERER U.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82